**Europäisches Patentamt**

**European Patent Office**

**Office européen des brevets**

(19)

(11) Veröffentlichungsnummer: **0 051 164**
**B1**

(12) **EUROPÄISCHE PATENTSCHRIFT**

(45) Veröffentlichungstag der Patentschrift:
14.12.83

(51) Int. Cl.³: **C 07 C 31/12**, C 07 C 29/04

(21) Anmeldenummer: **81108105.8**

(22) Anmeldetag: **09.10.81**

(54) **Verfahren zur kontinuierlichen Herstellung von sek.-Butylalkohol.**

(30) Priorität: **31.10.80 DE 3040997**

(43) Veröffentlichungstag der Anmeldung:
**12.05.82 Patentblatt 82/19**

(45) Bekanntmachung des Hinweises auf die Patenterteilung:
**14.12.83 Patentblatt 83/50**

(84) Benannte Vertragsstaaten:
**AT BE DE FR GB IT NL SE**

(56) Entgegenhaltungen:
**DE - B - 1 176 114**
**DE - B - 2 429 770**

(73) Patentinhaber: **DEUTSCHE TEXACO AKTIENGESELLSCHAFT, Überseering 40, D-2000 Hamburg 60 (DE)**

(72) Erfinder: **Neier, Wilhelm, Dr., An der Landwehr 40, D-4134 Rheinberg 3 (DE)**
Erfinder: **Webers, Werner, Rektor-Horn-Strasse 42, D-4134 Rheinberg 3 (DE)**
Erfinder: **Ruckhaber, Rainer, Zahnstrasse 56, D-4130 Moers 1 (DE)**
Erfinder: **Osterburg, Günther, Buchenstrasse 1, D-4100 Duisburg 17 (DE)**
Erfinder: **Ostwald, Wolf-Jürgen, Dr., Rheinkamper Strasse 35, D-4134 Rheinberg 3 (DE)**

(74) Vertreter: **Müller, Hans-Jürgen et al, Müller, Schupfner & Gauger Karlstrasse 5, D-2110 Buchholz/Nordheide (DE)**

### Verfahren zur kontinuierlichen Herstellung von sek.-Butylalkohol

Die Erfindung betrifft ein Verfahren zur kontinuierlichen Herstellung von im wesentlichen wasserfreiem sek.-Butylalkohol durch Hydratisierung von n-Butenen in Gegenwart stark saurer, in einem Festbett angeordneter Kationenaustauscher als Katalysator.

Aus DE-AS 24 29 770 ist ein Verfahren zur kontinuierlichen Herstellung von niederen Alkoholen an stark sauren Kationenaustauschern bekannt, bei dem der gebildete Alkohol dampfförmig zusammen mit dem überschüssigen Reaktionsgas am Kopf des Reaktors abgezogen wird und nach Entfernung des Gasanteils in Form eines über 80%igen Alkohols gewonnen wird. Nach diesem Verfahren kann der Alkohol sowohl durch Zwischenentspannung in einem Abscheidersystem wie in DE-AS 24 29 770 als auch durch Abtrennung in einer in bekannter Weise arbeitenden Druckkolonne (Stabilkolonne) gewonnen werden. In beiden Fällen wird z.B. sek.-Butylalkohol mit einem 15- bis maximal 23%igen Wasseranteil (je nach Gasquerschnittsbelastung) erhalten. Dieser Alkohol kann nicht ohne vorherige destillative Trocknung – nach herkömmlicher Art mit Benzol – dem Ketonisierungsreaktor zugeführt werden.

Gemäss DE-B-1 176 114 wird bei der Herstellung von tert.-Butanol aus iso-Buten in Gegenwart von dispergierten sulfonsäuregruppenhaltigen Kationenaustauschern das Reaktionsgemisch nach erfolgter Umsetzung bei Temperaturen von 50 bis 130 °C und Drücken zwischen 10 und 30 bar in einen Trennbehälter gegeben. Dort erfolgt Trennung in eine obere Kohlenwasserstoffschicht und eine untere wässrige Schicht, die die Hauptmenge Wasser und den Katalysator enthält. In der oberen organischen Phase befindet sich neben dem $C_4$-Schnitt das tert.-Butanol sowie noch beträchtliche Mengen Wasser, im Beispiel 6,8% Wasser bezogen auf tert.-Butanol.

Der vorliegenden Erfindung lag die Aufgabe zugrunde, das Verfahren zur Herstellung von sek.-Butylalkohol durch Hydratisierung von n-Butenen in einem Sumpfbett-Reaktor so durchzuführen, dass nach Abtrennung der Gasphase vom gebildeten Alkohol ein Rohalkohol gewonnen werden kann, der nur noch sehr geringe Anteile Wasser neben gebildetem Äther enthält. Ein derartiger Roh-sek.-Butylalkohol (ca. 99%ig) könnte ohne jede weitere Zwischendestillation einem Dehydrierungsreaktor zur Erzeugung von Methyläthylketon zugeführt werden.

Gegenstand der Erfindung ist ein Verfahren, bei dem der durch Direkthydratation hergestellte dampfförmige Alkohol am Kopf des Reaktors zusammen mit dem überschüssigen Reaktionsgas entnommen, unter Druck, 2 bis 60 bar, insbesondere 15 bis 40 bar, abgekühlt und dabei verflüssigt wird. Überraschenderweise wurde gefunden, dass bei Temperaturen zwischen 10 und 135 °C, vorzugsweise 80 bis 120 °C, bis zu 90% des in der überkritischen Gasphase gelösten Wassers abgeschieden wird, wobei der gelöste Alkohol fast quantitativ in der Gasphase verbleibt. Nach destillativer Abtrennung des Reaktionsgases von Alkohol in einer Druckkolonne wird am Sumpf der Kolonne ein Rohalkohol gewonnen, der deutlich weniger als 0,1% Wasser enthält. Das Verfahren der Erfindung ist energiesparend, denn der gewonnene Rohalkohol kann ohne weitere aufwendige destillative Aufbereitung sofort einem Dehydrierungsreaktor zugeführt werden.

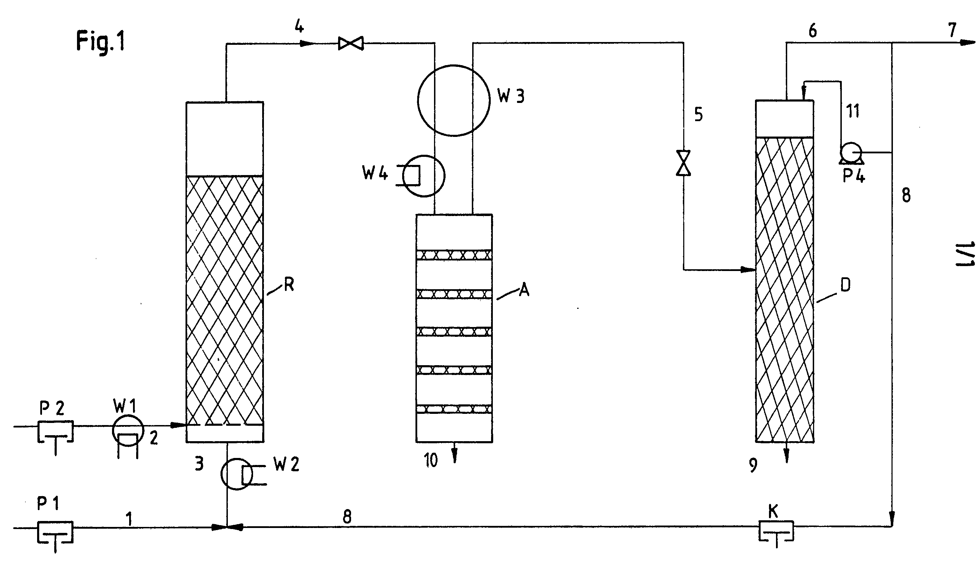

Das Verfahren der Erfindung wird anhand der schematischen Darstellung Abb. 1 beispielsweise beschrieben. Mit Hilfe der Dosierpumpe P 1 wird das Einsatzgas über Leitung 1 mit dem Kreislauf-Olefin aus Leitung 8 vermischt, im Verdampfer W 2 verdampft und am Sumpf über Leitung 3 in den Reaktor R geleitet. Mit der Pumpe P 2 wird das Reaktionswasser über Leitung 2 nach Vorheizen im Wärmeaustauscher W 1 dem Reaktor zugeführt. Der Reaktor ist mit einem stark sauren Ionenaustauscher auf Styrol-Divinylbenzolbasis gefüllt. Beide Reaktionskomponenten werden von unten nach oben durch das Reaktorbett geleitet. Am oberen Ende des Reaktors kann eventuell ein kleiner Wasserstrom entnommen werden (nicht gezeigt). Am Reaktorkopf wird der gesamte gebildete Alkohol dampfförmig mit dem überschüssigen Reaktionsgas (n-Butan/n-Butene) über Leitung 4 abgezogen, unter gleichem oder vermindertem Druck abgekühlt und verflüssigt und über Wärmetauscher W 3 und W 4 durch einen mit Demistergeweben ausgestatteten Abscheider A geleitet. Das abgeschiedene Wasser, das nur geringe Teile des Alkohols (< 1%) enthält, wird über Leitung 10 abgezogen und als Prozesswasser über Leitung 2 erneut eingesetzt. Das flüssige Alkohol-Butene/Butan-Gemisch wird durch Gegentausch in Wärmetauscher W 3 wieder verdampft und über Leitung 5 in einer Druckkolonne D in Alkohol (Leitung 9) und Reaktionsgas (Leitung 6) getrennt. Mit der Pumpe P 4 wird ein kleiner Rückfluss 11 zur Kolonne gegeben. Der grösste Teil des Reaktionsgases wird mit Hilfe des Kompressors K der Reaktion über Leitung 8 erneut zugeführt. Über Leitung 7 wird ein kleiner Restgasstrom entnommen.

Gemäss der vorliegenden Erfindung kann durch Abkühlung des den Reaktor verlassenden Produktgasstromes unter Druck auf 135 °C oder niedrigere Temperaturen der grösste Teil des mit dem Gas ausgetragenen Wassers abgeschieden werden. Bei der anschliessenden destillativen Abtrennung des Restgases, im Falle der Herstellung von sek.-Butylalkohol bei der Abtrennung von Butan/Butene-Gemisch, erhält man einen Alkohol mit einem Wassergehalt von deutlich unter 0,1%, wie 0,005 bis 0,05%. Dieser Alkohol kann ohne jede weitere Behandlung direkt zur Ketonisierung eingesetzt werden.

Ein Vorteil der Zwischenkühlung bzw. Zwischenkondensation liegt auch darin, dass man das Rücklaufverhältnis in der Stabilkolonne von 1,3 auf 0,3 absenken kann. Hierdurch wird eine Energieeinsparung an dieser Kolonne von etwa 43% erzielt. Da der Energieverbrauch dieser Kolonne einen wesentlichen Teil des Energiebedarfs des Gesamtverfahrens ausmacht, ist die Energieeinsparung an dieser Kolonne von grosser Bedeutung.

Die Abkühlung kann auch bei tieferen Temperaturen, etwa − 10 °C oder − 20 °C, erfolgen. Allerdings ist dies wesentlich aufwendiger und dürfte in der Regel aus wirtschaftlichen Gründen nicht zweckmässig sein.

Vorzugsweise erfolgt die Abkühlung des Produktgasstromes sowie dessen Verflüssigung einerseits und die Erwärmung sowie Verdampfung des den Abscheider verlassenden flüssigen Alkohols/Gas-Gemisches gleichzeitig mit Hilfe eines Wärmetauschers, des Wärmetauschers W 3.

Die folgenden Beispiele erläutern das erfindungsgemässe Verfahren (Beispiel 3) gegenüber Verfahren des Standes der Technik, die einerseits mit zwei Abscheidern arbeiten gemäss DE-AS 24 29 770 (Beispiel 1) und andererseits nur mit einer Druckkolonne (Beispiel 2):

Beispiel 1 (Stand der Technik)

Ein lotrecht angeordneter Rohrreaktor aus Edelstahl und mit 26 mm lichtem Durchmesser und 3 m Länge wurde bis zu einer Standhöhe von 2,83 m mit 1,5 l Raschig-Ringen Edelstahl, 4 × 4 mm) und danach bis zur gleichen Standhöhe mit 1,2 l eines handelsüblichen, makroporösen Kationenaustauscherharzes (sulfoniertes Styrol/Divinylbenzol-Mischpolymerisat) in der H$^{(+)}$-Form gefüllt. Dieses Füllkörper/Katalysator-Festbett wurde durch unter- und oberhalb desselben angeordnete Edelstahl-Drahtnetze gehalten.

Am Boden des Rohrreaktors wurden stündlich 232 g eines 87%igen n-Butengemisches (3,6 Mol n-Butene) und 58 g (3,2 Mol) Wasser eingesetzt. Im Reaktor wurden durch eine Mantelbeheizung eine Temperatur von 150 °C und ein Druck von 70 bar aufrechterhalten.

Aus dem Gasraum am Kopf des Reaktors wurde das dampfförmige Umsetzungsprodukt abgeführt. Ein Teil davon wurde von einer Umwälzpumpe in den Reaktor zurückgeführt und dabei mit zurückgeführtem Restgas und Frischgas zu einem etwa 78 bis 81% n-Butene enthaltenden Mischgas vermischt.

Der andere Teil des dampfförmigen Umsetzungsproduktes wurde auf 25 bis 30 bar entspannt und in einen ersten Abscheider eingeleitet. Im ersten Abscheider fiel nach der Abtrennung von C$_4$-Restgas ein flüssiger Rohalkohol an, der in einem zweiten Abscheider auf Normaldruck entspannt wurde.

Das 76% n-Butene enthaltende Restgas (Gasumsatz bezogen auf Frischgas = 52,6%) wurde vom ersten Abscheider (Druckabscheider) zu einem Kompressor geführt, wo es auf 70 bar verdichtet und in den Reaktor zurückgeführt wurde, und zwar mit dem Kreislaufstrom und dem 87%-igen Frischgasstrom gemeinsam in Gestalt eines 78 bis 81% n-Butene enthaltenden Mischgases.

Dem Kreislauf wurde stündlich ein 95,2 g n-Butene (1,7 Mol) und 30,4 g n-Butan (0,52 Mol) enthaltendes 76%iges Restgas entnommen, im zweiten Abscheider auf Normaldruck entspannt und ausgeschleust. Im zweiten Abscheider fielen stündlich 140 g (1,9 Mol) sek.-Butylalkohol und 0,7 bis 1,4 g Di-sek.-Butyläther nach Entfernen der C$_4$-Anteile in Form eines 77- bis 89%igen Rohalkohols an, der noch 10–22% Wasser enthielt. Die Raum/Zeit-Ausbeute an sek.-Butylalkohol betrug 1,6 Mol pro Liter Katalysator und Stunde, die Selektivität mehr als 99%.

Beispiel 2 (Stand der Technik)

Der gemäss Beispiel 1 am Reaktorkopf abgezogene Produktgasstrom wurde nach Entspannung auf 8 bar in eine Druckkolonne geleitet und dort in sek.-Butylalkohol und überschüssiges Reaktionsgas getrennt. Das überschüssige Reaktionsgas wurde wie vorher dem Reaktor wieder zugeführt. Ein kleiner Teil davon wurde als Restgas entnommen. Unter den sonst gleichen Reaktionsbedingungen wurde die gleiche Katalysatorleistung und Selektivität erhalten. Der erhaltene Roh-Alkohol enthielt noch 15 bis 10% Wasser.

Beispiel 3 (Beispiel der Erfindung)

Das in den Beispielen 1 und 2 beschriebene Verfahren wurde so geändert, dass der am Reaktorkopf zusammen mit dem überschüssigen Reaktionsgas abgezogene gebildete sek.-Butylalkohol unter einem Druck von 30 bar auf 100 °C abgekühlt und flüssig durch den mit Demistergeweben gefüllten Abscheider geleitet wurde. Der Abscheider bestand aus einem Druckrohr von 26 mm Durchmesser, der mit 6 Demistergewebeschichten je 50 mm Stärke gefüllt war. Zwischen den Demistergeweben war ein Freiraum von je 100 mm. Am Sumpf des Abscheiders konnte ca. 90% des vom überkritischen Reaktionsgas aus dem Reaktor ausgetragenen Wassers abgeschieden werden. Diese Wasserphase enthielt nur 0,6 bis 0,8% an sek.-Butylalkohol und Äther. Am Kopf des Abscheiders wurde ein Gemisch aus überschüssigem Reaktionsgas mit den Reaktionsprodukten sek.-Butylalkohol und Di-sek.-Butyläther abgezogen, im Gegentausch im Wärmeaustauscher W 3 verdampft und nach Entspannung auf 8 bar in eine Druckkolonne zur Trennung in Rohalkohol und Reaktionsgas geleitet. Über Leitung 8 wurde das überschüssige Reaktionsgas erneut komprimiert und dem Reaktor zugeleitet. Am Sumpf der Kolonne konnte ein Rohalkohol erhalten werden, der nur noch 0,01 bis 0,05% Wasser enthielt. Die Ausbeute und Selektivität war wie in Beispiel 1 angegeben.

**Patentansprüche**

1. Verfahren zur kontinuierlichen Herstellung von sek.-Butylalkohol durch katalytische Hydratation von n-Butenen in Gegenwart eines stark sauren Kationenaustauschers als Katalysator, der als Festbett angeordnet und von den Reaktionskomponenten bei einer Temperatur von 120 bis 180 °C unter einem Druck von 40 bis 200 bar von unten nach oben durchströmt wird, wobei das Molverhältnis Wasser zu Olefin 0,5 bis 10 und insbesondere 1,0 bis 3,0:1 ist, dadurch gekennzeichnet, dass der am Reaktorkopf abgezogene dampfförmige Produktstrom ggf. auf einen Druck von 2 bis 60 bar entspannt und unter diesem Druck auf eine Temperatur von 135 °C oder niedriger abgekühlt und dabei verflüssigt wird, in einem Abscheider in Wasser und flüssiges Alkohol-Butene/Butan-Gemisch aufgetrennt und das Alkohol-Butene/Butan-Gemisch unter Erwärmung verdampft, und anschliessend in einer Druckkolonne bei einem Druck von 3 bis 30 bar der Alkohol abgetrennt wird.

2. Verfahren nach Anspruch 1, dadurch gekennzeichnet, dass der Gasstrom unter Druck auf 10 bis 135 °C, vorzugsweise auf 80 bis 120 °C abgekühlt wird.

3. Verfahren nach Anspruch 1 oder 2, dadurch gekennzeichnet, dass im Abscheider ein Druck von 2 bis 60 bar, vorzugsweise 15 bis 40 bar eingehalten wird.

4. Verfahren nach einem der Ansprüche 1 bis 3, dadurch gekennzeichnet, dass mittels Wärmetausch der am Reaktorkopf abgezogene dampfförmige Produktstrom abgekühlt sowie verflüssigt und gleichzeitig das den Abscheider verlassende flüssige Alkohol-Butene/Butan-Gemisch erwärmt sowie verdampft wird.

## Revendications

1. Procédé pour la préparation en continu de l'acool secondaire butylique par hydratation catalytique de n-butènes en présence d'un échangeur de cations fortement acide à titre de catalyseur, cet échangeur de cations se trouvant sous forme d'un lit fixe qui est traversé du bas en haut par les composants de la réaction à une température de 120 à 180 °C et sous une pression de 40 à 200 bar, le rapport molaire eau/oléfine étant de 0,5 à 10 et plus particulièrement de 1,0 à 3,0:1, caractérisé en ce qu'on détend éventuellement le courant de produits gazeux retiré de la tête du réacteur à une pression de 2 à 60 bar et qu'on le refroidit sous cette pression à une température de 135 °C ou à une température inférieure à celle-ci, liquéfiant ainsi ledit courant qu'on sépare dans un séparateur en eau et en un mélange liquide alcool-butène/butane, ledit mélange alcool-butène/butane étant vaporisé par suite d'un chauffage, l'alcool étant alors séparé dans une colonne tenue sous une pression de 3 à 30 bar.

2. Procédé selon la revendication 1, caractérisé en ce qu'on refroidit le courant gazeux sous pression à une température de 10 à 135 °C, de préférence 80 à 120 °C.

3. Procédé selon les revendications 1 ou 2, caractérisé en ce qu'on maintient dans le séparateur une pression de 2 à 60 bar, de préférence 15 à 40 bar.

4. Procédé selon l'une des revendications 1 a 3, caractérisé en ce qu'on refroidit et liquéfie a l'aide d'un échange de chaleur le courant de produits trouvant sous forme de vapeur et provenant de la tête du réacteur le mélange liquide alcool-butène/butane quittant le séparateur étant simultanément chauffé et vaporisé.

## Claims

1. Process for the continuous production of secondary butyl alcohol by catalytic hydration of n-butenes in the presence of a strongly acidic cation exchanger as catalyst arranged as a fixed bed and flown through by the reaction components in upstream operation at a temperature of 120 °C to 180 °C and a pressure of 40 to 200 bar, the water/olefin mole ratio being 0.5 to 10.0., particularly 1.0 to 3.0:1 , characterized by optionally depressuring the vaporous product stream removed overhead from the reactor to 2 to 60 bar, cooling it at this pressure to a temperature of 135 °C or lower, and thus liquefying the product stream, separating it in a separator into water and liquid alcohol-butene/butane mixture and vaporizing the alcohol-butene/butane mixture under heat, and subsequently separating the alcohol in a pressurized column at a pressure of 3 to 30 bar.

2. Process according to claim 1, characterized by cooling the gas stream under pressure to 10 °C to 135 °C, preferably to 80 °C to 120 °C.

3. Process according to claim 1 or 2, characterized by maintaining in the separator a pressure of 2 to 60 bar, preferably 15 to 40 bar.

4. Process according to any one of claims 1 to 3, characterized by cooling and liquefying by heat exchange the vaporous product stream removed overhead from the reactor and simultaneously heating and vaporizing the liquid alcohol-butene/butane mixture leaving the separator.

Fig.1

0 051 164

1/1